# EUROPEAN PATENT SPECIFICATION

(11) **EP 4 069 867 B1**
(45) Date of publication and mention of the grant of the patent: **27.08.2025**
(21) Application number: 20895028.7
(22) Date of filing: 16.11.2020
(51) Int. Cl.: C12Q 1/6823, B01L 3/00

(54) **MAGNETIC CAPTURE BEAD MEDIATED MOLECULAR BARCODING OF NUCLEIC ACID TARGETS IN SINGLE PARTICLES AND COMPOSITIONS FOR USE IN THE SAME**
DURCH MAGNETISCHE EINFANGKÜGELCHEN VERMITTELTE MOLEKULARE BARCODIERUNG VON NUKLEINSÄUREZIELEN IN EINZELNEN PARTIKELN UND ZUSAMMENSETZUNGEN ZUR VERWENDUNG IN DERSELBEN
CODE À BARRES MOLÉCULAIRE À MÉDIATION PAR BILLES DE CAPTURE MAGNÉTIQUE DE CIBLES D'ACIDE NUCLÉIQUE DANS DES PARTICULES UNIQUES ET COMPOSITIONS DESTINÉES À ÊTRE UTILISÉES DANS CE DERNIER

(30) Priority: 04.12.2019 US 201962943647 P
(43) Date of publication of application: 12.10.2022
(73) Proprietor: Becton, Dickinson and Company, Franklin Lakes, NJ 07417-1880 (US)
(72) Inventor: SPUHLER, Philipp Stefan, San Carlos, California 94070 (US)
(74) Representative: Arnold & Siedsma
(86) International application number: PCT/US2020/060692
(87) International publication number: WO 2021/113065

(56) References cited:
- WO-A2-2007/106579
- AU-A1- 2016 364 722
- JP-B2- 5 054 096
- US-A1- 2002 119 470
- US-A1- 2014 371 097
- US-A1- 2016 053 253
- US-A1- 2016 289 669
- US-A1- 2018 267 036
- US-A1- 2019 276 820

## Description

### INTRODUCTION

The ability to detect and quantify specific nucleic acid and protein molecules in individual cells is critical for understanding the role of cellular diversity in development, health, and disease. Flow cytometry has become a standard technology for high-throughput detection of protein markers on single cells and has been widely adopted in basic research and clinical diagnostics. In contrast, nucleic acid measurements such as mRNA expression are typically conducted on bulk samples, obscuring the contributions from individual cells.

In order to characterize the complexity of cellular systems, it is highly desirable to develop methods, devices, and systems for monitoring the expression of large numbers of genes across many thousands of cells. Current technology allows measurement of gene expression of single cells in a massively parallel manner (e.g., >10,000 cells) by attaching cell specific oligonucleotide barcodes to poly(A) mRNA molecules from individual cells as each of the cells is co-localized with a barcoded reagent bead in a compartment.

In addition to cells, of increasing interest is the detection of nucleic acids in extracellular vesicles, e.g., exosomes and micro-vesicles. Extracellular vesicles, such as exosomes and micro-vesicles, are shed by virtually all cell types and are widespread in blood and other fluids. Multiple research reports have demonstrated that various RNA species (including mRNA, miRNAs, and lncRNAs) entrapped within EVs can be transferred from donor to acceptor cells and interfere in gene expression in the latter. Turchinovich et al., "Trascriptome of Extracellular Vesicles: Sate-of-the-Art," Front Immunol. (2019) 10: 202. As such, characterization of extracellular vesicle nucleic acids is of interest.

US2016053253A1 in its abstract refers to 'methods and compositions for multiplexed single cell gene expression analysis. Some methods and compositions include the use of droplets and/or beads bearing unique barcodes such as unique molecular barcodes (UMI).'

### SUMMARY

While current technology allows measurement of gene expression of single cells in a massively parallel manner (e.g., >10,000 cells), the inventors have identified certain inadequacies with methods used to date. For example, with currently employed platforms it can be difficult to partition small cells and vesicles having low sedimentation rates. Furthermore, currently employed platforms may barcode and sequence many cells that are not of interest, resulting in inefficient resource use. Embodiments of the invention address these and other needs

The invention is defined by the appended claims.

The disclosure (not claimed) relates to a magnetic capture bead mediated method of molecular barcoding nucleic acid targets of a particle, such as a cell or extracellular vesicle. Aspects include: a) combining a sample comprising the particle with a magnetic capture bead comprising a capture moiety for the particle to produce a captured sample; b) partitioning captured particles of the captured sample using an applied magnetic field mediated partitioning protocol to produce partitioned captured particles, wherein the partitioned captured particles are in spatial proximity to bead bound barcode nucleic acids comprising target binding regions; and c) lysing the partitioned captured particles so that nucleic acid acids released therefrom bind to the target binding regions to produce captured nucleic acids. Also provided are compositions, e.g., magnetic capture beads, including barcoded magnetic beads, as well as device/systems and kits, that find use in practicing embodiments of the methods.

Aspects of the method (not claimed) include: a) combining a sample with a magnetic capture bead comprising a capture moiety for the particle of the sample to produce a captured sample; b) partitioning captured particles of the captured sample using an applied magnetic field mediated partitioning protocol to produce partitioned captured particles, wherein the partitioned captured particles are in spatial proximity to a bead bound barcode nucleic acids comprising a target binding region, e.g., an oligo dT domain, a gene specific domain or a random sequence domain; and c) lysing the partitioned captured particles so that nucleic acids released therefrom bind to the target binding regions of the bead bound barcode nucleic acids to produce captured nucleic acids. In some instances, the bead bound barcode nucleic acids are tethered to the magnetic capture bead, while in other instances, the bead bound barcode nucleic acids are tethered to a barcode bead that is distinct from the magnetic capture bead. In some instances, the partitioned capture particles are partitioned into microwells. In some instances, the capture moiety comprises a specific binding member, such as an antibody or binding fragment thereof. In some instances, the bead bound barcode nucleic acids, in addition to the target binding region, further comprise one or more of a cell label domain, a unique molecular index domain and a universal primer binding domain, e.g., where the bead bound barcode nucleic acids have the following structure: bead-5'-universal primer binding domain-cell label domain-unique molecular index domain-target binding region-3'. The method may be employed to barcode nucleic acids from a variety of particles, such as biological particles, e.g., cells as well as sub-cellular sized particles, e.g., extra cellular vesicles, platelets, vesicles, such as exosomes or micro-vesicles. In some instances, the methods further include separating captured nucleic acids from other constituents of the partitioned captured particles, e.g., by employing an applied magnetic field. In some instance, the method further comprises pooling the captured nucleic acids. In some instances, the method further comprises subjecting the captured nucleic acids to cDNA synthesis reaction conditions to produce first strand cDNA domain comprising capture nucleic acids. In some instances, the method further comprises producing an amplicon composition from the first strand cDNA domain comprising capture nucleic acids. In some instances, the amplicon composition is produced from the first strand cDNA domain comprising capture nucleic acids using one or more rounds of amplification such as in embodiments wherein the amplicon composition comprises a next generation sequencing (NGS) library. In some instances, the amplicon composition comprises NGS adapter comprising nucleic acids. In some instances, the methods further include sequencing the NGS library.

In some instances (not claimed), the method includes: a) combining a sample containing the particle with a magnetic capture bead comprising: i )barcode nucleic acids comprising a target binding region; and ii) a capture moiety that specifically binds to the particle, to produce a captured sample; b) partitioning captured particles of the captured sample into microwells using an applied magnetic field mediated partitioning protocol to produce partitioned captured particles; lysing the partitioned captured particles so that nucleic acid acids released therefrom bind to target binding regions of the barcode nucleic acids to produce captured nucleic acids; subjecting the captured nucleic acids to cDNA synthesis reaction conditions to produce first strand cDNA domain comprising capture nucleic acids; producing a NGS library from the first strand cDNA domain comprising capture nucleic acids; and sequencing the NGS library to sequence nucleic acids of the target particle.

In some instances, the method further includes employing oligonucleotide labeled cellular component binding reagents, e.g., in applications where detection, e.g., quantitation, of one of or more cellular components, e.g., surface proteins, is desired. Oligonucleotide labeled cellular component-binding reagents employed in such embodiments include a cellular component-binding reagent, e.g., antibody or binding fragment thereof, coupled to a cellular component-binding reagent specific oligonucleotide comprising an identifier sequence for the cellular component-binding reagent that the cellular component-binding reagent specific oligonucleotide is associated therewith. In such instances, the magnetic capture bead may include a nucleic acid configured to capture, e.g., specifically bind to, a domain of the cellular component-binding reagent specific oligonucleotide. In this way, protein expression may be assayed in conjunction with gene expression, e.g., where multi-ohmic analysis is desired, e.g., combined analysis of transcriptome and proteome.

Also provided (but not claimed) are magnetic capture beads that find use in of the method. The magnetic capture beads include magnetic beads having stably associated therewith barcode nucleic acids and a capture moiety that specifically binds to a particle. In some instances, the capture moiety comprises an antibody or binding fragment thereof. In some instances, the barcode nucleic acids, in addition to a target binding region (e.g., an oligo dT domain, a gene specific domain or a random sequence domain), further include one or more of a cell label domain, a unique molecular index domain and a universal primer binding domain, e.g., where the bead bound barcode nucleic acids have the following structure: bead-5'-universal primer binding domain-cell label domain-unique molecular index domain-target binding region-3'.

Specifically, a magnetic capture bead according to the present disclosure comprises barcode nucleic acids tethered to the magnetic capture bead, wherein the barcode nucleic acids comprise a target binding region; and a capture moiety that specifically binds to a particle that can be lysed to release nucleic acids that bind to the target binding regions.

Also provided (but not claimed) is a device that find use in embodiments of the methods. The device includes: a) a substrate comprising 100 or more microwells that comprise a magnetic capture bead comprising: i) barcode nucleic acids comprising a target binding region; and ii) a capture moiety that specifically binds to a particle; and b) a flow cell in fluid communication with the substrate. In some instances, the capture moiety comprises an antibody or binding fragment thereof. In some instances, the barcode nucleic acids, in addition to a target binding region (e.g., an oligo dT domain, a gene specific domain or a random sequence domain), further include one or more of a cell label domain, a unique molecular index domain and a universal primer binding domain, e.g., where the bead bound barcode nucleic acids have the following structure: bead-5'-universal primer binding domain-cell label domain-unique molecular index domain-target binding region-3'.

Specifically the disclosure includes a device comprising a substrate comprising 100 or more microwells that comprise a magnetic capture bead comprising, barcode nucleic acids tethered to the magnetic capture bead, wherein the barcode nucleic acids comprise a target binding region; and a capture moiety that specifically binds to a particle that can be lysed to release nucleic acids that bind to the target binding regions, and a flow cell in fluid communication with the substrate.

Also provided is a system (not claimed) that finds use in the method. The system include: a) a substrate comprising 100 or more microwells that comprise a magnetic capture bead comprising: i) barcode nucleic acids comprising a target binding region; and ii) a capture moiety that specifically binds to a particle; b) a flow cell in fluid communication with the substrate; and c) a flow controller; wherein the flow controller is configured to control the delivery of fluids to the flow cell; and in some instances a magnetic field applicator, e.g., as described in greater detail below. In some instances, the capture moiety comprises an antibody or binding fragment thereof. In some instances, the barcode nucleic acids, in addition to a target binding region (e.g., an oligo dT domain, a gene specific domain or a random sequence domain), further include one or more of a cell label domain, a unique molecular index domain and a universal primer binding domain, e.g., where the bead bound barcode nucleic acids have the following structure: bead-5'-universal primer binding domain-cell label domain-unique molecular index domain-target binding region-3'.

Specifically the disclosure includes a system comprising a substrate comprising 100 or more microwells that comprise a magnetic capture bead comprising barcode nucleic acids tethered to the magnetic capture bead, wherein the barcode nucleic acids comprise a target binding region; and a capture moiety that specifically binds to a particle that can be lysed to release nucleic acids that bind to the target binding regions, a flow cell in fluid communication with the substrate; and a flow controller; wherein the flow controller is configured to control the delivery of fluids to the flow cell.

Also provided is a kit that find use the method. The kit includes: a magnetic capture bead comprising barcode nucleic acids tethered to the magnetic capture bead, wherein the barcode nucleic acids comprise a target binding region, and wherein the magnetic capture bead also comprises a capture moiety that specifically binds to a target particle that can be lysed to release nucleic acids that bind to the target binding regions. In some instances, the capture moiety comprises an antibody or binding fragment thereof. In some instances, the barcode nucleic acids, in addition to a target binding region (e.g., an oligo dT domain, a gene specific domain or a random sequence domain), further include one or more of a cell label domain, a unique molecular index domain and a universal primer binding domain, e.g., where the bead bound barcode nucleic acids have the following structure: bead-5'-universal primer binding domain-cell label domain-unique molecular index domain-target binding region-3'. In some instances, the kit further includes a device comprising: (i) a substrate comprising 100 or more microwells; and (ii) a flow cell in fluid communication with the substrate.

### BRIEF DESCRIPTION OF THE DRAWINGS

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.
FIGS. 1A and 1B provide representations of magnetic capture beads according to various embodiments of the invention.
FIG. 2 provides a representation of captured sample preparation, according to an embodiment of the invention.
FIGS. 3A and 3B provide a presentation of partitioning captured particles, in accordance with an embodiment of the invention.
FIGS. 4A to 4C provide a presentation of partitioning captured particles, in accordance with an embodiment of the invention.
FIG. 5 provides a representation of a workflow that employs a system according to an embodiment of the invention.
FIG. 6 provides an illustration of a sequencing library preparation workflow that may be employed in embodiments of the invention.

### DEFINITIONS

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adapter" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adapters can be linear. The adapters can be pre-adenylated adapters. The adapters can be double- or single-stranded. One or more adapters can be located on the 5' or 3' end of a nucleic acid. When the adapters comprise known sequences on the 5' and 3' ends, the known sequences can be the same or different sequences. An adapter located on the 5' and/or 3' ends of a polynucleotide can be capable of hybridizing to one or more oligonucleotides immobilized on a surface. An adapter can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adapters can comprise identical and/or universal nucleic acid sequences and the 3' adapters can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adapters (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein, an antibody can be a full-length (e.g., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment. In some embodiments, an antibody is a functional antibody fragment. For example, an antibody fragment can be a portion of an antibody such as F(ab')2, Fab', Fab, Fv, sFv and the like. An antibody fragment can bind with the same antigen that is recognized by the full-length antibody. An antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (for example, CD8, CD34, and CD45), and therapeutic antibodies.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" can be used interchangeably. It is understood from the disclosure that if a molecule can hybridize to another molecule it may be the complement of the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequence-able label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein n is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from a RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adapter primer," "universal primer adapter" or "universal adapter sequence" are used interchangeably to refer to a nucleotide sequence that can be used to hybridize to barcodes (e.g., stochastic barcodes) to generate gene-specific barcodes. A universal adapter sequence can, for example, be a known sequence that is universal across all barcodes used in methods of the disclosure. For example, when multiple targets are being labeled using the methods disclosed herein, each of the target-specific sequences may be linked to the same universal adapter sequence. In some embodiments, more than one universal adapter sequences may be used in the methods disclosed herein. For example, when multiple targets are being labeled using the methods disclosed herein, at least two of the target-specific sequences are linked to different universal adapter sequences. A universal adapter primer and its complement may be included in two oligonucleotides, one of which comprises a target-specific sequence and the other comprises a barcode. For example, a universal adapter sequence may be part of an oligonucleotide comprising a target-specific sequence to generate a nucleotide sequence that is complementary to a target nucleic acid. A second oligonucleotide comprising a barcode and a complementary sequence of the universal adapter sequence may hybridize with the nucleotide sequence and generate a target-specific barcode (e.g., a target-specific stochastic barcode). In some embodiments, a universal adapter primer has a sequence that is different from a universal PCR primer used in the methods of this disclosure.

### OVERVIEW OF MOLECULAR BARCODING OF NUCLEIC ACID TARGETS IN A SINGLE CELL

The methodology underlying the methods, devices, and systems of the present disclosure utilizes a deposition strategy to implement single cell, molecular barcoding assays for large numbers of individual cells. For example, molecular targets from individual cells can be stochastically labeled with a cellular label (also referred to as a cellular index, barcode, or tag) and a molecular label (also referred to as a molecular index, barcode, or tag) by associating individual cells with individual barcoded beads barcode nucleic acids tethered thereto, wherein each individual barcoded bead comprises a plurality of attached stochastic labels. The stochastic labels attached to a given bead can be used to randomly label protein or nucleic acid targets from an associated cell. In some embodiments, single cells are randomly distributed into a plurality of microwells (e.g., of a micro well array). A combinatorial library of barcoded beads, each comprising a plurality of tethered stochastic barcode nucleic acid labels, is also randomly distributed into the plurality of microwells so that a subset of the microwells contains both a single cell and a single bead. In some embodiments the barcoded beads are deposited prior to depositing the cells. In other embodiments the barcoded beads are deposited after depositing the cells. In embodiments, e.g., where the barcoded beads include a capture moiety (e.g., as described in greater detail below) for a target cell, the beads and cells (or subcellular particle, e.g., vesicle) may be deposited at the same time, e.g., as a bound complex of the bead and the cell. The stochastic labels comprising the cellular and molecular barcodes may further comprise a target recognition region that is capable of attaching to or hybridizing with molecular targets, for example, nucleic acid molecules. The target molecules can be released from each cell, for example by lysing the cell, and then attached to or hybridized with the stochastic labels on a corresponding barcoded bead. In some embodiments the target molecules are released from the cells by cleavage, e.g. enzymatic cleavage. In some embodiments, e.g., when the target molecules are mRNA molecules, the beads are retrieved from the microwells following hybridization of the mRNA target molecules to the stochastic labels, and pooled prior to performing reverse transcription, amplification, and sequencing reactions.

In some embodiments, the plurality of stochastic labels attached to a given bead comprises a cellular label that is identical for all of the stochastic labels attached to the bead, while the cellular labels for the pluralities of stochastic labels attached to different beads are different. In some embodiments, the plurality of stochastic labels attached to a given bead comprises a diverse set of molecular labels selected from a set comprising a specified number of unique molecular label sequences. In some embodiments, the plurality of stochastic labels attached to a given bead may comprise the same target recognition region. In some embodiments, the plurality of stochastic labels attached to a given bead may comprise two or more different target recognition regions.

In some embodiments, the bead library has a cellular label diversity (i.e. a number of unique cellular label sequences) that is at least one or two orders of magnitude higher than the number of cells to be labeled, such that the probability that each cell is paired with a unique cell barcode is very high. For example, the probability that each cell is paired with a unique cell barcode may be greater than 80%, greater than 90%, greater than 95%, greater than 99%, greater than 99.9%, greater than 99.99%, or greater than 99.999%.

In some embodiments, the molecular label diversity (i.e. the number of unique molecular label sequences) for the plurality of stochastic labels attached to a bead is at least one or two orders of magnitude higher than the estimated number of occurrences of a target molecule species to be labeled, such that the probability that each occurrence of a target molecule (e.g. an mRNA molecule) within a cell becomes uniquely labeled is also very high. For example, the probability that each occurrence of a target molecule is paired with a unique molecular barcode may be greater than 80%, greater than 90%, greater than 95%, greater than 99%, greater than 99.9%, greater than 99.99%, or greater than 99.999%. In these embodiments, the number of occurrences of a target molecule species in each cell may be counted (or estimated) by determining the number of unique molecular label sequences that are attached to the target molecule sequence. In many embodiments, the determining step may be performed through sequencing of an amplified library of labeled target molecules (or their complementary sequences).

In some embodiments, the molecular label diversity for the plurality of stochastic labels attached to a bead is comparable to or low compared to the estimated number of occurrences of a target molecule species to be labeled, such that there is a significant probability that the multiple occurrences of a given type of target molecule will be labeled by more than one copy of a given molecular label. In these embodiments, the number of target molecules in each cell may be calculated from the number of unique molecular label sequences attached to the target molecule sequence with the use of Poisson statistics.

In certain embodiments, the target molecules of interest are mRNA molecules expressed within a single cell. Since cDNA copies of all or a portion of the polyadenylated mRNA molecules in each cell are covalently archived on the surface of a corresponding bead, any selection of gene transcripts may be subsequently analyzed. A digital gene expression profile for each cell may be reconstructed when the barcoded transcripts are sequenced and assigned to the cell of origin (based on the cellular label identified) and counted (based on the number of unique molecular labels identified). An exemplary description of the assay methodology can be found in Fan, et ah, "Combinatorial Labeling of Single Cells for Gene Expression Cytometry", Science 347(6222):628; and Science 347(6222): 1258367.

Various elements of the above overview are now reviewed in greater detail.

### Barcodes and Digital Counting

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding such as the Precise^{™} assay (Cellular Research, Inc. (Palo Alto, CA)) can correct for bias induced by PCR and library preparation steps by using molecular labels (MLs) to label mRNAs during reverse transcription (RT).

The Precise^{™} assay can utilize a non-depleting pool of stochastic barcodes with large number, for example 6561 to 65536, unique molecular labels on poly(T) oligonucleotides to hybridize to all poly(A)-mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate, a droplet or other partition, can be pooled into a single tube for PCR amplification and sequencing, e.g., via a next generation sequencing protocol, e.g., as described below. Raw sequencing data can be analyzed to produce the number of reads, the number of stochastic barcodes with unique molecular labels, and the numbers of mRNA molecules.

Barcoding, such as stochastic barcoding, has been described in, for example, Fu et al., Proc Natl Acad Sci U.S.A., 2011 May 31,108(22):9026-31; U.S. Patent Application Publication No. US2011/0160078; Fan et al., Science, 2015 February 6, 347(6222):1258367; US Patent Application Publication No. US2015/0299784; PCT Application Publication No. WO2015/031691. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be a mRNA species comprising mRNA molecules with identical or nearly identical sequence, an antibody identifier sequence, etc. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs and/or cellular component-binding reagent specific oligonucleotide, etc. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

### Universal Labels

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer, and may therefore be referred to as a universal primer binding domain. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

### Dimension Labels

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as 10⁶ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least, or be at most, 1, 2, 3, 4, 5, 10, 15, 20, 25, 30, 35, 40, 45, 50, 100, 200, or 300, nucleotides in length. A dimension label can comprise between about 5 to about 200 nucleotides. A dimension label can comprise between about 10 to about 150 nucleotides. A dimension label can comprise between about 20 to about 125 nucleotides in length.

### Spatial Labels

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., a well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as 10⁶ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell, and therefore may be referred to as a cell label domain. In some embodiments, the cell label is identical for all barcodes attached to a given solid support (e.g., a bead), but different for different solid supports (e.g., beads). In some embodiments, the percentage of barcodes on the same solid support comprising the same cell label can be, or be about 60%, 70%, 80%, 85%, 90%, 95%, 97%, 99%, 100%, or a number or a range between any two of these values. In some embodiments, the percentage of barcodes on the same solid support comprising the same cell label can be, or be about 60%, 70%, 80%, 85%, 90%, 95%, 97%, 99%, or 100%. For example, at least 60% of barcodes on the same solid support can comprise the same cell label. As another example, at least 95% of barcodes on the same solid support can comprise the same cell label.

There can be as many as 10⁶ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, 10², 10³, 10⁴, 10⁵, 10⁶, 10⁷, 10⁸, or 10⁹, unique barcode sequences. The unique molecular label sequences can be attached to a given solid support (e.g., a bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, 10², 10³, 10⁴, 10⁵, 10⁶, 10⁷, 10⁸, or 10⁹, unique molecular label sequences. Barcodes with unique molecular label sequences can be attached to a given solid support (e.g., a bead).

For stochastic barcoding using a plurality of stochastic barcodes, the ratio of the number of different molecular label sequences and the number of occurrence of any of the targets can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about, 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

### Target-Binding Region

A barcode can comprise one or more target binding regions, such as capture probes (which may also be referred to as capture nucleic acids). In some embodiments, a target-binding region can hybridize with a target of interest. In some embodiments, the target binding regions can comprise a nucleic acid sequence that hybridizes specifically to a target (e.g., a target nucleic acid, a target molecule, a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, a poly(dA) sequence, a poly(dT) sequence, a poly(dG) sequence, a poly(dC) sequence, or a combination thereof. For example, the target binding region can be an oligo(dT) sequence (e.g., an oligo dT domain) that hybridizes to the poly(A) tail on mRNA molecules. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

A random multimer sequence can be, for example, a random dimer, trimer, quatramer, pentamer, hexamer, septamer, octamer, nonamer, decamer, or higher multimer sequence of any length (wherein such instances the target binding region may be referred to as a random sequence domain).

In some embodiments, the target binding region is the same for all barcodes attached to a given bead. In some embodiments, the target binding regions for the plurality of barcodes attached to a given bead can comprise two or more different target binding sequences. A target binding region can be, or be about, 1, 2, 3, 4, 5, 10, 15, 20, 25, 30, 35, 40, 45, 50, or a number or a range between any two of these values, nucleotides in length. A target binding region can be at most about 1, 2, 3, 4, 5, 10, 15, 20, 25, 30, 35, 40, 45, 50 or more nucleotides in length.

In some embodiments, a target-binding region can comprise an oligo(dT) (i.e., e.g., an oligo dT domain) which can hybridize with mRNAs comprising polyadenylated ends. A target-binding region can be gene-specific. For example, a target-binding region can be configured to hybridize to a specific region of a target (e.g., where the target binding region is a gene specific domain).

A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a barcode comprises a gene-specific target-binding region, the barcode can be referred to herein as a gene-specific barcode.

The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

### Universal Adapter Primer

A barcode can comprise one or more universal adapter primers. For example, a gene-specific barcode, such as a gene-specific stochastic barcode, can comprise a universal adapter primer. A universal adapter primer can refer to a nucleotide sequence that is universal across all barcodes. A universal adapter primer can be used for building gene-specific barcodes. A universal adapter primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adapter primer can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adapter primer can be from 5-30 nucleotides in length.

### Linker

When a barcode comprises more than one of a type of label (*e.g.,* more than one cell label or more than one barcode sequence, such as one molecular label), the labels may be interspersed with a linker label sequence. A linker label sequence can be at least about 5, 10, 15, 20, 25, 30, 35, 40, 45, 50 or more nucleotides in length. A linker label sequence can be at most about 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

### Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (Pleasanton CA). In some implementation, a gel bead can comprise a polymer-based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be degradable. For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of Fe₃O₄ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, a number or a range between any two of these values, or more. In some embodiments, the number of cell label sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality of oligonucleotides on each particle can comprise different barcode sequences (e.g., molecular labels). In some embodiments, the number of barcode sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. In some embodiments, the number of barcode sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometers. In some embodiments, the diameter of the bead can be, or be about, 0.1, 0.5, 1, 2, 3, 4, 5, 6, 7, 8, 9, 10, 20, 30, 40, 50 micrometers, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, or be about, 10%, 20%, 30%, 40%, 50%, 60%, 70%, 80%, 90%, 100%, 150%, 200%, 250%, 300%, or a number or a range between any two of these values, longer or shorter than the diameter of the cell. In some embodiments, the diameter of the beads can be at least, or be at most, 10%, 20%, 30%, 40%, 50%, 60%, 70%, 80%, 90%, 100%, 150%, 200%, 250%, or 300% longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic. A bead can be hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

### DETAILED DESCRIPTION

Embodiments of the invention provide magnetic capture bead mediated methods of molecular barcoding nucleic acid targets of a particle, such as a cell or extracellular vesicle. Aspects of the methods (not claimed) include: a) combining a sample comprising the particle with a magnetic capture bead comprising a capture moiety for the particle to produce a captured sample; b) partitioning captured particles of the captured sample using an applied magnetic field mediated partitioning protocol to produce partitioned captured particles, wherein the partitioned captured particles are in spatial proximity to bead bound barcode nucleic acids comprising target binding regions; and c) lysing the partitioned captured particles so that nucleic acids released therefrom, and optionally cellular component-binding reagent specific oligonucleotides released therefrom, bind to the target binding regions to produce captured nucleic acids. Also provided are compositions, e.g., magnetic capture beads, including barcoded magnetic beads, as well as device/systems and kits, that find use in practicing embodiments of the methods.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

In further describing various aspects of the invention, various protocols and reagents/systems used therein are reviewed first in greater detail, followed by an in-depth review of the various methods of the invention as well as a description of embodiments of kits that find use in practicing various embodiments of the methods.

### METHODS

As reviewed above, magnetic bead mediated methods of molecular barcoding of target nucleic acids from a particle, such as a cell or subcellular component, e.g., vesicle, are provided. Aspects of the methods include combining a sample at least suspected of including a particle of interest with one or more magnetic capture beads (including a library of magnetic capture beads), where the magnetic capture bead includes a capture moiety for the particle to produce a captured sample. The sample which is contacted with the one or more magnetic capture beads, may be any sample, e.g., a sample that includes one or more cells, as well as other components, e.g., extracellular components, such as vesicles. In some embodiments, the sample can be a particle, e.g., single cell or extracellular vesicle (e.g., micro-vesicle, exosome or apoptotic body), a plurality of particles, e.g., cells and/or vesicles, a tissue sample, a tumor sample, a blood sample, or the like. In some embodiments, the sample can comprise a mixture of cell types, such as normal cells, tumor cells, blood cells, B cells, T cells, maternal cells, fetal cells, etc., or a mixture of cells from different subjects, as well as subcellular components thereof, e.g., vesicles. In some instances, the sample is an aqueous sample that includes one or more types of particles, e.g., cells and/or vesicles, such as cells and other components of sub-cellular size, e.g., vesicles, are present. While the number of particles in a given aqueous sample employed in workflow embodiments of the invention may vary, in some instances the number of particles in a given sample volume ranges from 0.0001 to 10 billion particles /ml. Where the target particles are rare, e.g., circulating tumor cells (CTCs), fetal cells in maternal blood, infectious bacterial or fungal cells, the number of particles in a given sample may ranging , such as 0.0001 to 100 particles/ml, such as 0.01 to 10 particles/ml. Where the target particles are abundant, e.g., EVs, RBCs, the number of particles in a given volume may range from 10 million to 10 billion particles/ml, such as 100 million to 10 billion particles/ml. The above provided concentrations may be lower or higher depending on dilution or concentrating of the sample. The given concentrations represent naturally occurring concentrations in a sample.

In some embodiments, the methods include barcoding nucleic acids from cells. In some instances, the methods include barcoding nucleic acids from sub-cellular sized particles, e.g., extracellular vesicles, where such sub-cellular size particles may have diameters that are 1000 nm or less. In some embodiments, the methods include barcoding nucleic acids of an extracellular vesicle, where in some instances the extracellular vesicle has a diameter of 5µ or less, such as 1µ or less, where in some instances the extracellular vesicle has a diameter ranging from 30 to 2500 nm, such as 30 to 1000 nm. In some instances, the particle is a micro vesicle (e.g., having a diameter ranging from 100 to 100 nm). In some instances, the particle is an exosome (e.g., having a diameter ranging from 30 to 150 nm).

The sample, e.g., as described above, is contacted with one or more magnetic capture beads comprising a capture moiety for the particle, e.g., cell or subcellular (such as vesicle) component of the sample. The magnetic capture beads are beads that are influenced (i.e., moved) in space by application of a magnetic field. As such, they can be moved in space, e.g., moved from one location to another, by application of a magnetic field, e.g., from another magnet, to the environment of the magnetic capture bead. The magnetic capture bead comprises a magnetic solid support having stably associated therewith, e.g., on a surface thereof, a capture moiety for a particle, e.g., a cell or subcellular sample component, e.g., vesicle. The bead can comprise one or more types of solid, porous, or hollow sphere, ball, bearing, cylinder, or other similar configuration to which a capture moiety can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape. As the beads are magnetic, they can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), and the like. The beads may also include a number of additional materials, e.g., ceramic, plastic, glass, polystyrene, silica, methylstyrene, acrylic polymers, titanium, latex, Sepharose, agarose, hydrogel, polymer, cellulose, nylon, or any combination thereof.

Stably associated with the solid support of the magnetic capture bead is a capture moiety for the particle, e.g., cell or vesicle. The capture moiety is a moiety that binds to a component (i.e., determinant), such as a surface protein or other structure, of the particle. In the broadest sense the binding may be any kind of binding, including specific or non-specific binding. In some instances, the capture moiety specifically binds to a determinant, such as a surface protein, of the particle. Where the capture moiety specifically binds to a determinant of the particle, the capture moiety and the determinant have affinity for each other. The affinity between a pair of a specific binding member and the determinant to which it specifically binds may vary, where in some instances they may specifically bind to each other in a binding complex that is characterized by a KD (dissociation constant) of 10⁻⁵ M or less, 10⁻⁶ M or less, 10⁻⁷ M or less, 10⁻⁸ M or less, 10⁻⁹ M or less, 10⁻¹⁰ M or less, 10⁻¹¹ M or less, 10⁻¹² M or less, 10⁻¹³ M or less, 10⁻¹⁴ M or less, or 10⁻¹⁵ M or less (it is noted that these values can apply to other specific binding pair interactions mentioned elsewhere in this description, in certain embodiments). Any suitable determinant binding reagents may be employed as capture moieties, such as protein binding reagents, antibodies or fragments thereof, aptamers, small molecules, ligands, peptides, oligonucleotides, etc., or any combination thereof. For example, a capture moiety can comprise an antibody, for example, an antibody specific for a specific moiety (e.g., receptor) on a target, e.g., cell or vesicle. The antibody can be a full-length (i.e., naturally occurring or formed by normal immunoglobulin gene fragment recombinant processes) immunoglobulin molecule (e.g., an IgG antibody) or an immunologically active (i.e., specifically binding) portion of an immunoglobulin molecule, like an antibody fragment. The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

As mentioned above, the capture moiety is stably associated with a surface of the solid support of the magnetic capture bead. By stably associated is meant that the capture moiety is immobilized relative to the surface location of the solid support to which it is attached. As used herein, the terms "immobilized", "tethered," and "attached," are used interchangeably, and can refer to covalent or non-covalent means for attaching capture moieties to a solid support.

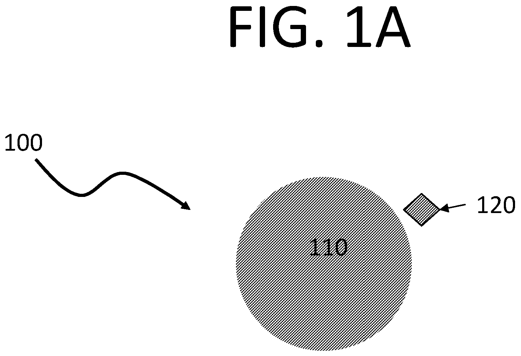

An example of a magnetic capture that include a capture moiety is illustrated in FIG. 1A. As illustrated in FIG. 1A, magnetic capture bead 100 includes a magnetic bead 110 (e.g., as described above) having stably associated with a surface thereof a capture moiety 120. As reviewed above, the capture moiety may vary, where examples of capture moieties include, but are not limited to: antibodies specific to the target particles (e.g., cells or extracellular vesicles), biotin, avidin, or another molecule (e.g., that binds to antibody that is conjugated with avidin, biotin, or other molecule complementary to the capture moiety stably associated with the bead).

Optionally, the capture magnetic bead may also include barcode nucleic acids, e.g., as described above, associated therewith. When present, the barcode nucleic acids include a target binding region. As reviewed above, the target binding region can comprise a nucleic acid sequence that hybridizes specifically to a target (e.g., a target nucleic acid, a target molecule, a cellular nucleic acid to be analyzed), for example to a specific gene sequence, such as a gene specific domain. In some embodiments, a target binding region can comprise a nucleic acid sequence that can attach (e.g., hybridize) to a specific location of a specific target nucleic acid. In other instances, the target binding region may be non-specific, e.g., an olio dT domain. In yet other instances, a target binding region may be random, such as a random hexamer, e.g., where the target binding region is a random sequence domain.

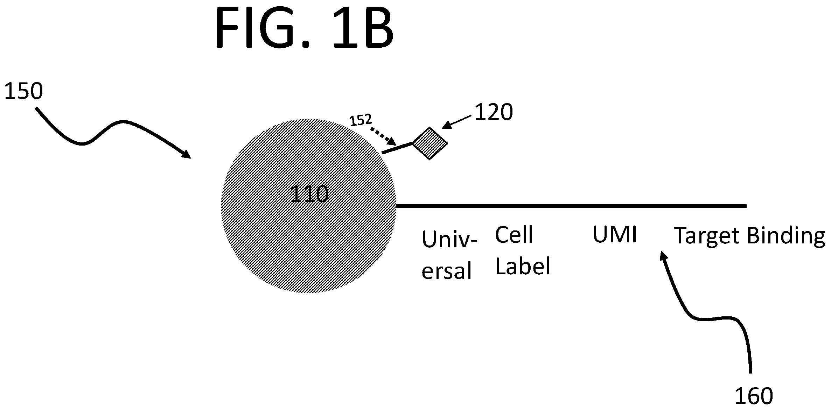

Examples of magnetic capture beads that include barcode nucleic acids are shown in FIG. 1B. As illustrated in FIG. 1B, magnetic capture bead 150 includes a magnetic bead 110 (e.g., as described above) having stably associated with a surface thereof a capture moiety 120, which is tethered to the surface of the bind by a linker 152. Also tethered to the surface of magnetic bead 110 is bead bound barcode nucleic acid 160. Bead bound barcode nucleic acid has the following structure: bead (110)-5'-universal primer binding domain (162)-cell label domain(164)-unique molecular index domain (166)-target binding region(168)-3', where these domains are as described above.

As reviewed above, the sample is contacted with a magnetic capture bead, including a plurality of magnetic capture beads, such as library of magnetic capture beads, to produce a captured sample. The number of different beads to which a given sample is contacted in any given protocol may vary, where in some instances the number is 1 or more, such as 10 or more, 50 or more, 100 or more, 1,000 or more, 5,000 or more, 10,000 or more, including 20,000 or more. The magnetic beads to which a given sample is contacted may be identical or different from each other, e.g., in terms of capture moiety or other component, such as barcode, such that the population, e.g., library, of capture magnetic beads to which the sample is contact includes a plurality of distinct beads that differ from other by some feature, such as barcode, capture moiety, etc.

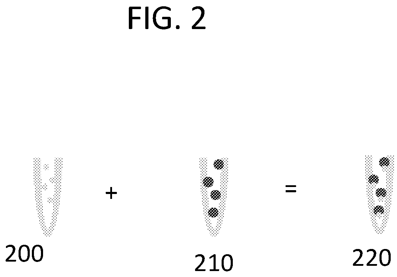

The sample and beads are contacted with each other under conditions sufficient for the capture moiety of the beads to bind to its particle to produce a captured sample that includes binding complexes of particles, e.g., cells, vesicles, etc., bound to magnetic capture beads. The conditions may vary, so long as the desired binding complexes are produced. The resultant captured sample includes captured particles that are binding complexes made up of particles, e.g., cells, vesicles, etc., bound to magnetic capture beads. An example of preparation of a captured sample is illustrated in FIG. 2. As shown in FIG. 2, sample 200 is combined with magnetic capture beads 210 to produce the captured sample, 220, which is made up of binding complexes of particles, e.g., cells, vesicles, etc., bound to magnetic capture beads. Following preparation of the capture sample, optionally the captured particles may be separated from other constituents of the captured sample, e.g., non-bound particles, unbound beads, etc.

Following production of the captured sample, aspects of the methods include partitioning captured particles of the captured sample using an applied magnetic field mediated partitioning protocol to produce partitioned captured particles. By partitioning is meant that the captured particles are placed into small reaction chambers, which may be fluidically isolated structures defined by solid materials, such as microwells, configured to accommodate the captured particles. In some embodiments of the disclosed methods, devices, and systems, a plurality of microwells that are randomly distributed across a substrate are used. In some embodiments, the plurality of microwells are distributed across a substrate in an ordered pattern, e.g. an ordered array. In some embodiments, a plurality of microwells are distributed across a substrate in a random pattern, e.g., a random array. The microwells can be fabricated in a variety of shapes and sizes. Appropriate well geometries include, but are not limited to, cylindrical, elliptical, cubic, conical, hemispherical, rectangular, or polyhedral, e.g., three dimensional geometries comprised of several planar faces, for example, rectangular cuboid, hexagonal columns, octagonal columns, inverted triangular pyramids, inverted square pyramids, inverted pentagonal pyramids, inverted hexagonal pyramids, or inverted truncated pyramids. In some embodiments, non-cylindrical microwells, e.g. wells having an elliptical or square footprint, may offer advantages in terms of being able to accommodate larger cells. In some embodiments, the upper and/or lower edges of the well walls may be rounded to avoid sharp corners and thereby decrease electrostatic forces that may arise at sharp edges or points due to concentration of electrostatic fields. Thus, use of rounded off corners may improve the ability to retrieve beads from the microwells. Microwell dimensions may be characterized in terms of of absolute dimensions. In some instances, the average diameter of the microwells may range from about 5 µm to about 100 µm. In other embodiments, the average microwell diameter is at least 5 µm, at least 10 µm, at least 15 µm, at least 20 µm, at least 25 µm, at least 30 µm, at least 35 µm, at least 40 µm, at least 45 µm, at least 50 µm, at least 60 µm, at least 70 µm, at least 80 µm, at least 90 µm, or at least 100 µm. In yet other embodiments, the average microwell diameter is at most 100 µm, at most 90 µm, at most 80 µm, at most 70 µm, at most 60 µm, at most 50 µm, at most 45 µm, at most 40 µm, at most 35 µm, at most 30 µm, at most 25 µm, at most 20 µm, at most 15 µm, at most 10 µm, or at most 5 µm. The volumes of the microwells used in the methods of the invention may vary, ranging in some instances from from about 200 µm³ to about 800,000 µm³. In some embodiments, the micro well volume is at least 200 µm³, at least 500 µm³, at least 1,000 µm³, at least 10,000 µm³, at least 25,000 µm³, at least 50,000 µm³, at least 100,000 µm³, at least 200,000 µm³, at least 300,000 µm³, at least 400,000 µm³, at least 500,000 µm³, at least 600,000 µm³, at least 700,000 µm³, or at least 800,000 µm³. In other embodiments, the microwell volume is at most 800,000 µm³, at most 700,000 µm³, at most 600,000 µm³, 500,000 µm³, at most 400,000 µm³, at most 300,000 µm³, at most 200,000 µm³, at most 100,000 µm³, at most 50,000 µm³, at most 25,000 µm³, at most 10,000 µm³, at most 1,000 µm³, at most 500 µm³, or at most 200 µm³. The number of microwells in a given device employed in embodiments of the invention may vary, where in some instances the number is 100 or more, such as 250 or more, e.g., 500 or more, including 1000 or more, such as 5,000 or more, e.g., 10,000 or more, wherein some instances the number is 15,000 or less, e.g., 12,500 or less. Microwells suitable for use in embodiments of the invention are further described in PCT application serial no. PCT/US2016/014612 published as WO/2016/118915.

In partitioning the captured sample, the captured particles may be positioned in microwells using any convenient protocol. The disclosure provides for methods for contacting the captured sample to partitions in order to partition the sample. A captured sample comprising, for example, can be introduced into structures, e.g., microwells, to partition the sample. The captured sample can be contacted, for example, by gravity flow wherein captured particles can settle into the partitioning structures. In some instances, the captured sample is contacted with, e.g., by flowing it across, the microwells such that captured particles are deposited into the microwells. The captured sample may be flowed through a flow cell in fluidic communication with the microwells. Suitable protocols and systems for partitioning the capture particles into microwells are described in Microwells suitable for use in embodiments of the invention are further described in PCT application serial no. PCT/US2016/014612.

In partitioning the capture sample, the captured sample is contacted with the microwells using an applied magnetic field, e.g., where the captured sample is flowed across the microwells while a magnetic field is applied to the captured sample. The applied magnetic field is one that moves the captured particles towards the microwells from the captured sample and depending on it orientating and the nature of the magnetic capture particles may be attractive or repulsive to the magnetic capture particles. For example, where the magnetic field is applied from beneath the bottom of the microwells, the magnetic field may be attractive to the magnetic capture particles. Alternatively, where the magnetic field is applied from above the microwells, the magnetic field may be repulsive to the magnetic capture particles. The magnetic field may be applied using any convenient source, e.g., a permanent magnetic, an electromagnet, etc. While the strength of the applied magnetic field may vary as desired, in some instances the strength ranges from 10 to 15,000 Gauss, such as 100 to 1000 Gauss. A system and methods for using the same that include one or more magnets and controllers therefor which may be employed in embodiments of the invention is described in PCT application serial no. PCT/US2016/014612.

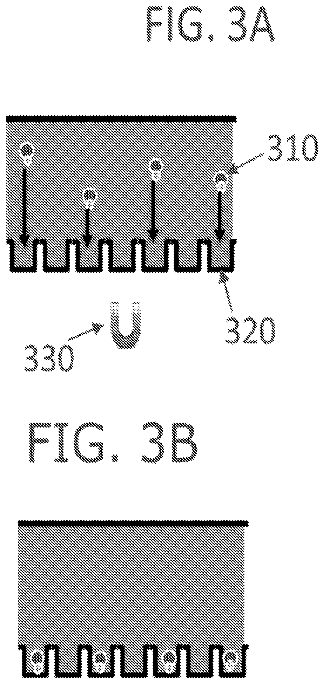

FIG. 3A provides a schematic representation of partitioning captured particles prepared from magnetic capture beads that include bead bound barcode nucleic acids, e.g., as illustrated in FIG. 1B, in microwells using a flow cell. As illustrated in FIG. 3A, the captured particles 310 that include particles, e.g., cells or extracellular vesicles, bound to magnetic capture beads that include bead bound barcode nucleic acids are flowed over an array of microwells 320 under the influence of applied electric field (as illustrated by magnet 330), which results in partitioning of the captured particles into the wells, as shown in FIG. 3B.

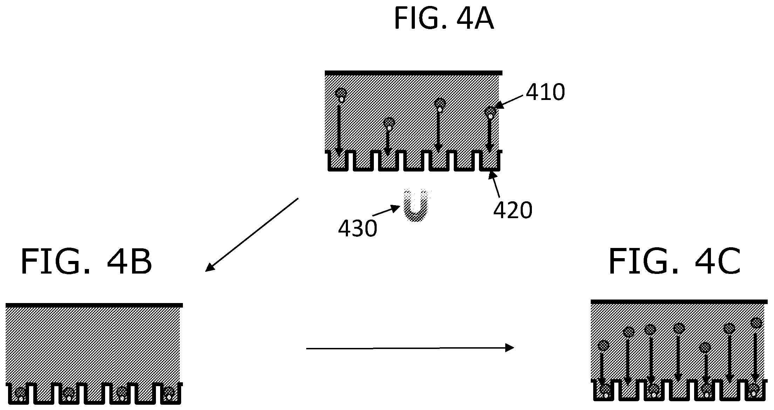

FIG. 4A provides a schematic representation of partitioning captured particles prepared from magnetic capture beads that to not include bead bound barcode nucleic acids, e.g., as illustrated in FIG. 1A, in microwells using a flow cell. As illustrated in FIG. 4A, the captured particles 410 that include particles, e.g., cells or extracellular vesicles, bound to magnetic capture beads that do not include bead bound barcode nucleic acids are flowed over an array of microwells 420 under the influence of applied electric field (as illustrated by magnet 430), which results in partitioning of the captured particles into the wells, as shown in FIG. 4B. Next, beads that includes barcode nucleic acids are partitioned into the microwells, as shown in FIG. 4C, such that certain of the wells include both captured particles and barcoded beads.

Partitioning of the captured sample, e.g., as described above, results in partitioned captured particles that are in spatial proximity to a bead bound barcode nucleic acids that include a target binding region, e.g., as described above. In some instances, the bead bound barcode nucleic acids are stably associated with the magnetic capture bead. In such instances, the partitions, e.g., microwells, may only include the captured particle, since the bead component of the captured particle includes the barcode nucleic acids. In other instances, the bead bound barcode nucleic acids are part of a separate bead that is distinct from the magnetic capture bead, e.g., a barcoded bead such as described above. In such instances, the partitions, e.g., microwells, may include both the captured particle and a separate barcoded bead that includes the barcode nucleic acids. When barcodes are in close proximity to targets of the captured particles, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

Following the partitioning of the particles, e.g., cells and/or vesicles, as described above, the particles can be lysed to liberate the target molecules so that the released target molecules, e.g., nucleic acids, can bind to the target binding regions of the barcode nucleic acids to produced captured nucleic acids. Particle lysis can be accomplished by any of a variety of means, for example, by chemical or biochemical means, by osmotic shock, or by means of thermal lysis, mechanical lysis, or optical lysis. Particles can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9, 10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

Following lysis of the particles and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule, e.g., when subject to DNA synthesis reaction conditions to produce first strand cDNA domain comprising capture nucleic acids.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

In some instances, the methods further include employing oligonucleotide labeled cellular component binding reagents, e.g., in applications where detection, e.g., quantitation, of one of or more cellular components, e.g., surface proteins, is desired. Oligonucleotide labeled cellular component-binding reagents employed in such embodiments include a cellular component-binding reagent, e.g., antibody or binding fragment thereof, coupled to a cellular component-binding reagent specific oligonucleotide comprising an identifier sequence for the cellular component-binding reagent that the cellular component-binding reagent specific oligonucleotide is associated therewith. In such instances, the magnetic capture bead may include a nucleic acid configured to capture, e.g., specifically bind to, a domain of the cellular component-binding reagent specific oligonucleotide. In this way, protein expression may be assayed in conjunction with gene expression, e.g., where multi-ohmic analysis is desired, e.g., combined analysis of transcriptome and proteome. In such instances, the methods may include preparing the captured sample with oligonucleotide labeled cellular component binding reagents, and then provide for capture of cellular component-binding reagent specific oligonucleotides released from the capture, partitioned cells. Further details regarding use of oligonucleotide labeled cellular component-binding reagents are found in United States Published Patent Application Nos. US20180267036 and US20200248263.

In some instances, the method further comprises separating captured nucleic acids from other constituents of the partitioned captured particles. The retrieval of solid support-based collections of attached target-barcode molecules can be implemented by use of magnetic beads and an externally-applied magnetic field.

Where desired, a given workflow may include a pooling step where a product composition, e.g., made up of captured nucleic acids, synthesized first strand cDNAs or synthesized double stranded cDNAs, is combined or pooled with product compositions obtained from one or more additional samples, e.g., particles, such as cells and or vesicles. In some instances, the pooling step is performed just after hybridization step between barcoded nucleic acids and target nucleic acids, e.g., as reviewed above. The number of different product compositions produced from different samples, e.g., cells, that are combined or pooled in such embodiments may vary, where the number ranges in some instances from 2 to 1,000,000, such as 3 to 200,000, including 4 to 100,000 such as 5 to 50,000, where in some instances the number ranges from 100 to 10,000, such as 1,000 to 5,000. Prior to or after pooling, the product composition(s) can be amplified, e.g., by polymerase chain reaction (PCR), such as described in greater detail below.

Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

The disclosure provides for a method to create a target-barcode conjugate using any convenient protocol, such as reverse transcription or nucleotide extension (e.g., of a sample indexing oligonucleotide or a cellular component=binding reagent specific oligonucleotide). The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of an mRNA molecule to generate to a labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, a target is a cDNA molecule. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

One or more nucleic acid amplification reactions can be performed to create multiple copies of the labeled target nucleic acid molecules. Amplification can be performed in a multiplexed manner, wherein multiple target nucleic acid sequences are amplified simultaneously. The amplification reaction can be used to add sequencing adapters to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of pafindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids. As such, methods may include producing an amplicon composition from the first strand cDNA domain comprising capture nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adapters to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adapters can be added for sequencing using, for example, next generation sequencing (NGS). The sequencing results can be used to determine cell labels, molecular labels, and sequences of nucleotide fragments of the one or more copies of the targets

In certain embodiments, the methods provided further include subjecting a prepared expression library, e.g., an amplicon composition produced as described above, to an NGS protocol. The protocol may be carried out on any suitable NGS sequencing platform. NGS sequencing platforms of interest include, but are not limited to, a sequencing platform provided by Illumina^{®} (e.g., the HiSeq^{™}, MiSeq^{™} and/or NextSeq^{™} sequencing systems); Ion Torrent^{™}

(e.g., the Ion PGM^{™} and/or Ion Proton^{™} sequencing systems); Pacific Biosciences (e.g., the PACBIO RS II Sequel sequencing system); Life Technologies^{™} (e.g., a SOLiD sequencing system); Oxford Nanopore (e.g., Minion), Roche (e.g., the 454 GS FLX+ and/or GS Junior sequencing systems); or any other sequencing platform of interest. The NGS protocol will vary depending on the particular NGS sequencing system employed. Detailed protocols for sequencing, e.g., which may include further amplification (e.g., solid-phase amplification), sequencing the amplicons, and analyzing the sequencing data are available from the manufacturer of the NGS sequencing system employed.

### DEVICES AND SYSTEMS

Disclosed (but not claimed) herein are devices and systems that find use in practicing embodiments of the invention. In some embodiments, the devices include: a) a substrate comprising: i) at least 100 microwells, and ii) a plurality of captured particles and/or barcoded beads, wherein a plurality of the at least 100 microwells each contain a single captured particle optionally with a single barcoded bead (e.g., when the magnetic capture bead does not include barcoded nucleic acids), and b) a flow cell in fluid communication with the substrate. In some embodiments, also present are at least one inlet port and at least one outlet port, wherein the at least one inlet port and at least one outlet port are in fluid communication with the flow cell via fluid channels, wherein the at least one inlet port and at least one outlet port are capable of directing a flow of a fluid through the flow cell, thereby contacting the microwells with the fluid. In some embodiments, the devices further comprising a valve that prevents fluid flow within the device unless a pipette tip is inserted into the conical feature of the pipette tip interface.

Also disclosed (but not claimed) herein are systems comprising: a) a device comprising: i) a substrate comprising at least 100 microwells; ii) a flow cell in fluid communication with the substrate; and iii) at least one inlet port and at least one outlet port, wherein the at least one inlet port and at least one outlet port are capable of directing a flow of a fluid through the flow cell, thereby contacting the microwells with the fluid; and b) a flow controller; wherein the flow controller is configured to control the delivery of fluids.

In some embodiments, the systems further comprise fluids wherein the fluids comprise particles (e.g., cell and/or vesicle) samples, bead suspensions, assay reagents, or any combination thereof. In some embodiments, the device is a removable, consumable component of the system. In some embodiments, cell samples and bead suspensions are dispensed or injected directly into the device by the user. In some embodiments, beads and assay reagents other than cell samples are preloaded in the device. In some embodiments, the flow controller is configured to intersperse fluid injections into the flow cell with air injections. In some embodiments, the systems further comprise a distribution mechanism for enhancing the uniform distribution of cells and beads across the at least 100 microwells, wherein the distribution mechanism performs an action selected from the group consisting of rocking, shaking, swirling, recirculating flow, low frequency agitation, and high frequency agitation, or any combination thereof. In some embodiments, the systems further comprise a cell lysis mechanism, e.g., that uses a high frequency piezoelectric transducer for sonicating the cells. In some embodiments, the systems further comprise a temperature controller for maintaining a user-specified temperature, or for ramping temperature between two or more specified temperatures over two or more specified time intervals. In some embodiments, the systems further comprise a magnetic field controller for creating magnetic field gradients used in eluting beads from the at least 100 microwells or for transporting beads through the device. In some embodiments, the systems further comprise an imaging system configured to capture and process images of all or a portion of the at least 100 microwells, wherein the imaging system further comprises an illumination subsystem, an imaging subsystem, and a processor. In some embodiments, the imaging system is configured to perform bright- field, dark- field, fluorescence, or quantitative phase imaging. In some embodiments, the imaging system is configured to provide real-time image analysis capability, and wherein the real-time image analysis is used to control a distribution mechanism for enhancing the uniform distribution of cells or beads across the at least 100 microwells so that a predetermined cell or bead distribution is achieved. In some embodiments, the predetermined distribution of cells and beads is one in which at least 10% of the microwells contain both a single cell and a single bead. In some embodiments, the predetermined distribution of cells and beads is one in which at least 25% of the microwells contain both a single cell and a single bead. In some embodiments, the systems further comprise a selection mechanism, wherein information derived from the processed images is used to identify a subset of cells exhibiting one or more specified characteristics, and the selection mechanism is configured to either include or exclude the subset of cells from subsequent data analysis. In some embodiments, the selection mechanism comprises physical removal of beads co-localized with cells of the identified subset of cells from the at least 100 microwells. In some embodiments, the selection mechanism comprises physical entrapment of beads co-localized with cells of the identified subset of cells in the at least 100 microwells. In some embodiments, the selection mechanism comprises use of dual-encoded beads wherein each individual bead is both optically-encoded and encoded by an attached oligonucleotide cellular label, and sequencing of the cellular labels attached to beads co-localized with cells of the identified subset of cells generates a list of sequence data to be included or excluded from further analysis. In some embodiments, the flow controller is configured to deliver a first test compound to the at least 100 microwells at a first time, and to deliver a cell lysis reagent to the at least 100 microwells at a second time. In some embodiments, the first time and the second time are the same. In some embodiments, the one or more specified characteristics are selected from the group consisting of cell size, cell shape, live cells, dead cells, a specified range of intracellular pH, a specified range of membrane potential, a specified level of intracellular calcium, the presence of one or more specified cell surface markers, and the expression of one or more specified genetic markers.

Also present may be software residing in a computer readable medium programmed to perform one or more of the following sequence data analysis steps: a) decoding or demultiplexing of sample barcode, cell barcode, molecular barcode, and target sequence data; b) automated clustering of cellular labels to compensate for amplification or sequencing errors, wherein the sequence data is collected for a library of stochastically-labeled target oligonucleotide molecules; c) alignment of sequence data with known reference sequences; d) determining the number of reads per gene per cell, and the number of unique transcript molecules per gene per cell; e) statistical analysis to predict confidence intervals for determinations of the number of transcript molecules per gene per cell; and f) statistical analysis to cluster cells according to gene expression data or to identify subpopulations of rare cells.

Also present may be software residing in a computer readable medium programmed to perform the following image processing and instrument control steps: a) detection of microwells in one or more images of a plurality of microwells; b) detection of microwells containing single cells in one or more images of a plurality of microwells; c) detection of microwells containing two or more cells in one or more images of a plurality of microwells, and determining the number of cells in each of the detected microwells; d) detection of microwells containing single beads in one or more images of a plurality of microwells; e) detection of microwells containing two or more beads in one or more images of a plurality of microwells, and determining the number of beads in each of the detected microwells; f) determining the number of microwells containing single cells after performing step (b); g) determining the number of microwells containing single beads after performing step (d); and h) determining the number of microwells containing single cells and single beads after performing steps (b) and (d), wherein the numbers determined in steps (f) - (h) are used to control an apparatus configured to distribute cells and beads across a plurality of microwells.

Also present may be software residing in a computer readable medium programmed to perform the following image processing and instrument control steps: a) detection of a subset of cells exhibiting one or more specified characteristics in one or more images of a plurality of microwells, wherein a fraction of the microwells contain cells; b) determining the locations of the microwells which contain the subset of cells; and c) using the locations determined in step (b) to control a selection apparatus configured to exclude the subset of cells from subsequent sequence data analysis.

In some embodiments, the selection apparatus of step c) is configured to include only the subset of cells in subsequent sequence data analysis. In some embodiments, the one or more images of a plurality of microwells are images selected from the group consisting of bright-field images, dark-field images, fluorescence images, luminescence images, and phosphorescence images. In some embodiments, the software further comprises the use of one or more algorithms selected from the group consisting of the Canny edge detection method, the Canny-Deriche edge detection method, the Sobel operator method, a first-order gradient detection method, a second order differential edge detection method, a phase coherence edge detection method, an intensity thresholding method, an intensity clustering method, an intensity histogram-based method, the generalized Hough transform, the circular Hough transform, the Fourier transform, the fast Fourier transform, wavelet analysis, and auto-correlation analysis. In some embodiments, the one or more specified characteristics are selected from the group consisting of cell size, cell shape, live cells, dead cells, a specified range of intracellular pH, a specified range of membrane potential, a specified level of intracellular calcium, the presence of one or more specified cell surface markers, and the expression of one or more specified genetic markers.

A system and methods for using the same such as described above which may be employed in embodiments of the invention is described in PCT application serial no. PCT/US2016/014612 published as WO/2016/118915

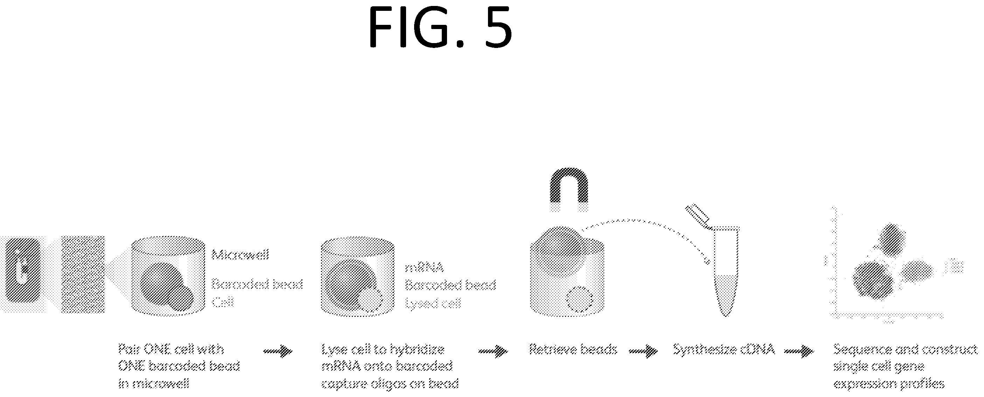

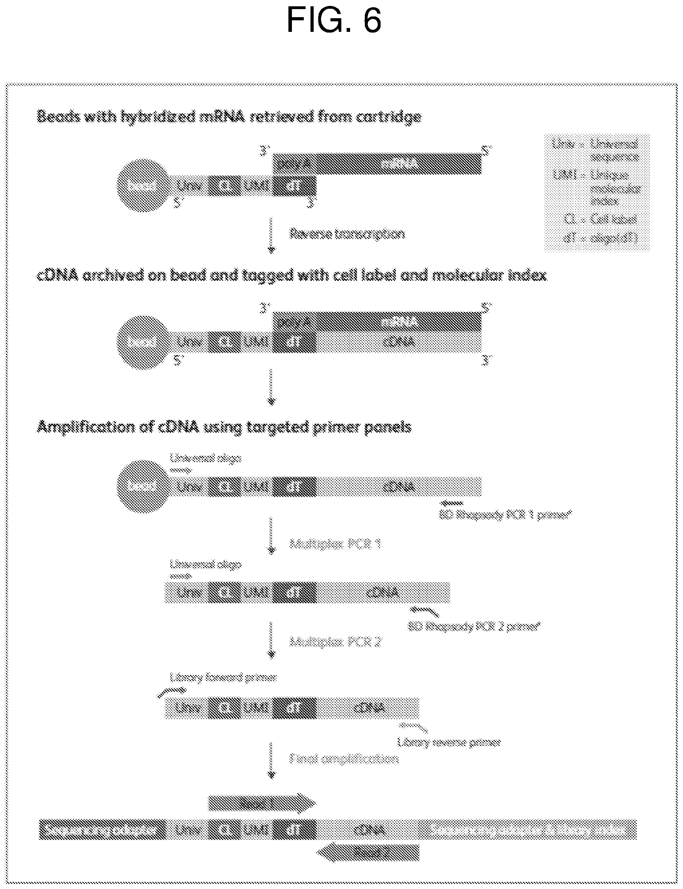

An example of a workflow according to an embodiment of the invention using a system such as described above that is commercially available as the Rhapsody^{™} Single-Cell Analysis System (Becton, Dickinson and Company) is illustrated in FIG. 5. As shown in FIG. 5, captured particles made up of a cell bound to a magnetic capture bead that also includes bead bound barcode nucleic acids is partitioned into a microwell of an array using an applied magnetic field, e.g., as illustrated in FIGS. 3A and 3B. The cells of the partitioned captured particles are then lysed so that mRNA released from the cell hybridizes to barcoded capture nucleic acids on the bead. The bead is then retrieved from the wells using an applied magnetic field and employed to generate a sequencing library, e.g., as illustrated in FIG. 6, which is then sequenced, e.g., using a next generation sequencing platform, such as described above.

### FLOW CYTOMETRY

In some embodiments, the method comprises isolating, obtaining, and/or enriching cells of interest. Isolating, obtaining, and/or enriching cells of interest has been described in U.S. Patent Application Publication No. 2016/0244828. For example, the method comprises isolating, obtaining, and/or enriching cells of interest can be performed with a flow cytometer or flow cytometrically. In some embodiments, the flow cytometer utilizes fluorescence-activated cell sorting.

Flow cytometry is a valuable method for the analysis and isolation of cells. As such it has a wide range of diagnostic and therapeutic applications. Flow cytometry utilizes a fluid stream to linearly segregate cells such that they can pass, single file, through a detection apparatus. Individual cells can be distinguished according to their location in the fluid stream and the presence of detectable markers or moieties (such as fluorophores on oligonucleotides associated with cellular component binding reagents). Cells flow through the focused interrogation point where at least one laser directs a laser beam to a focused point within the channel. The sample fluid containing cells is hydrodynamically focused to a very small core diameter by flowing sheath fluid around the sample stream at a very high volumetric rate. The small core diameter can be fewer than 10, 20, 30, 40, 50, 60, 70, 80, 90, 100, 110, 120, 130, 140, 150, 160, 170, 180, 190, 200 micrometers, or a number or a range between any two of these values. The volumetric rate of the sheath fluid can be on the order of at least 10, 20, 30, 40, 50, 60, 70, 80, 90, 100, 200, 300, 400, 500, 600, 700, 800, 900, or 1000 times, or a number or a range between any two of these values, the volumetric rate of the sample. This results in very fast linear velocities for the focused cells on the order of meters per second. So each cell spends a very limited time in the excitation spot, for example fewer than 1, 2, 3, 4, 5, 6, 7, 8, 9, 10, 20, 30, 40, 50, 60, 70, 80, 90, 100 microseconds, or a number or a range between any two of these values. Once the cells pass the interrogation point the cells cannot be redirected to the interrogation point again because the linear flow velocity cannot be reversed.

Flow cytometers are analytical tools that enable the characterization of cells on the basis of optical parameters such as light scatter and fluorescence. In a flow cytometer, cells in a fluid suspension are passed by a detection region in which the cells are exposed to an excitation light, typically from one or more lasers, and the light scattering and fluorescence properties of the cells are measured. Cells or components thereof typically are labeled with fluorescent dyes to facilitate detection. A multiplicity of different cells or components can be simultaneously detected by using spectrally distinct fluorescent dyes to label the different cells or components. In some implementations, a multiplicity of photodetectors, one for each of the scatter parameters to be measured, and one for each of the distinct dyes to be detected are included in the analyzer. The data obtained comprise the signals measured for each of the light scatter parameters and the fluorescence emissions.

Isolation of biological cells has been achieved by adding a sorting or collection capability to flow cytometers. Cells in a segregated stream, detected as having one or more desired characteristics, are individually isolated from the sample stream by mechanical or electrical removal. This method of flow sorting has been used to sort cells of different types, to separate sperms bearing X and Y chromosomes for breeding, to sort chromosomes for genetic analysis, and to isolate particular organisms from complex biological populations.

A common flow sorting technique utilizes drop sorting in which a fluid stream containing linearly segregated cells is broken into drops and the drops containing cells of interest are electrically charged and deflected into a collection tube by passage through an electric field. Drop sorting systems are capable of forming drops at a rate of about 100, 500, 1000, 2000, 3000, 4000, 5000, 6000, 7500, 10000, 20000, 30000, 40000, 50000, 60000, 75000, 100000, 200000, 300000, 400000, 500000, 600000, 750000, 1000000 drops/second, or a number or a range between any two of these values, in a fluid stream that is passed through a nozzle having a diameter less than 1000, 750, 600, 500, 400, 300, 200, 100, 75, 60, 50, 40, 30, 20, 10, 5, 2, 1 micrometers, or a number or a range between any two of these values. Drop sorting requires that the drops break off from the stream at a fixed distance from the nozzle tip. The distance is normally on the order of a few millimeters from the nozzle tip and can be maintained for an unperturbed fluid stream by oscillating the nozzle tip at a predefined frequency.

The linearly segregated cells in the stream can be characterized as they pass through an observation point situated just below the nozzle tip. Once a cell is identified as meeting about 1, 2, 3, 4, 5, 6, 7, 8, 9, 10, 20, 30, 40, 50, 60, 75, 100, 200, 300, 400, 500, 600, 750 criteria, or a number or a range between any two of these values, or more than 1000 criteria, the time at which it will reach the drop break-off point and break from the stream in a drop can be predicted. Possibly, a brief charge is applied to the fluid stream just before the drop containing the selected cell breaks from the stream and then grounded immediately after the drop breaks off. The drop to be sorted maintains an electrical charge as it breaks off from the fluid stream, and all other drops are left uncharged. The charged drop is deflected sideways from the downward trajectory of the other drops by an electrical field and collected in a sample tube. The uncharged drops fall directly into a drain.

Cytometers can further comprise means for recording the measured data and analyzing the data. For example, data storage and analysis can be carried out using a computer connected to the detection electronics. For example, the data can be stored in tabular form, where each row corresponds to data for one cell, and the columns correspond to each of the measured parameters. The use of standard file formats, such as an "FCS" file format, for storing data from a flow cytometer facilitates analyzing data using separate programs and/or machines. Using current analysis methods, the data typically are displayed in 2-dimensional ("2D") plots for ease of visualization, but other methods can be used to visualize multidimensional data.

The parameters measured using a flow cytometer typically include the excitation light that is scattered by the cell along a mostly forward direction, referred to as forward scatter ("FSC"), the excitation light that is scattered by the cell in a mostly sideways direction, referred to as side scatter ("SSC"), and the light emitted from fluorescent molecules in one or more channels (range of frequencies) of the spectrum, referred to as FL1, FL2, etc., or by the fluorescent dye that is primarily detected in that channel. Different cell types can be identified by the scatter parameters and the fluorescence emissions resulting from labeling various cell proteins with dye-labeled antibodies.

Fluorescence-activated cell sorting or is a specialized type of flow cytometry. It provides a method for sorting a heterogeneous mixture of cells into two or more containers or wells of a microtiter plate, one cell at a time, based upon the specific light scattering and fluorescent characteristics of each cell. It records fluorescent signals from individual cells, and physically separates cells of particular interest. The acronym FACS is trademarked and owned by Becton Dickinson.

The cell suspension is placed near the center of a narrow, rapidly flowing stream of liquid. The flow is arranged so that on the average (Poisson distribution) there is a large separation between cells relative to their diameter. A vibrating mechanism causes the stream of cells to break into individual droplets. The system is adjusted so that there is a low probability of more than one cell being in a droplet. Just before the stream breaks into droplets the flow passes through one or more laser intersects where the fluorescent character of interest of each cell is measured. If a cell is to be collected, a charge is applied to the flow cell during the period of time one or more drops form and break off from the stream. These charged droplets then fall through an electrostatic deflection system that diverts droplets into target containers based upon the charge applied to the droplet.

Non-limiting examples of the sorting device suitable to use for cell sorting include a BD FACSJazz^{™} cell sorter, a BD FACSseq^{™} cell sorter, or any other cell sorters including a Bio-Rad Laboratories, Inc. (Hercules, CA) S3e^{™} Cell Sorter, a Sony Biotechnology Inc. (San Jose, CA) SH800 cell sorter, a Beckman Coulter Inc. (Brea, CA) MoFlo^{™} XDP cell sorter.

In some embodiments, the methods utilize the tagging and staining of cell surface markers for cell sorting. In some embodiments, the methods utilize the tagging of cell surface markers for magnetic depletion of cells not of interest, interfering cells, and debris. In some embodiments, the methods include one or more of determining genotype of the patient based on the obtained sequence information; determining phenotype of the patient based on the obtained sequence information; determining one or more genetic mutation of the patient based on the sequence information; and predicting susceptibility of a patient to one or more diseases. At least one of the one or more diseases is cancer or a hereditary disease.

FACS can sort cells based on the tagging and staining of cell surface markers using antibodies that target detectable cell markers. The antibodies include monoclonal and polyclonal antibodies that are coupled to fluorophores. Detectable cell markers include cell surface markers on cells of interest. In some embodiments, magnetic depletion can be based on the tagging of cell surface markers with magnetic beads with antibodies, including monoclonal antibodies and polyclonal antibodies, conjugated to their surfaces targeting cells not of interest, interfering cells, and/or debris. Non-limiting examples of cells surface markers include CD surface markers, growth factor/cytokine, chemokine receptors, nuclear receptors, and other receptors. Examples of cell surface markers include, but are not limited to, ALCAM; CD166; ASGR1; BCAM; BSG; CD147; CD14; CD19; CD2; CD200; CD127 BV421; CD25 BB515; CD161 PE; CD45RA PerCP-Cy^{™}5.5; CD15S AF647; CD4 APC-H; CD4; CD25; CD127; CD45RA; CD15S; CD161; CD3; EpCAM; CD44; and Her2/Neu. Examples of growth factors/cytokines, chemokine receptors include ACVR 1B; ALK4; ACVR2A; ACVR2B; BMPR1A; BMPR2; CSF1R; MCSFR; CSF2RB; EGFR; EPHA2; EPHA4; EPHB2; EPHB4; and ERBB2. Examples of nuclear receptors include androgen receptor; CAR; ER Alpha; ER Beta; ESRRA; ESRRB; ESRRG; FXR; Glucocorticoid Receptor; LXR-a; LXR-b; PPARA; PPARD; PPARG; PXR; SXR; Estrogen Receptor Beta; Progesterone Receptor; RARA; RARB; RARG; RORA; RXRA; RXRB; THRA; THRB; and Vitamin D3 Receptor. Examples of other receptors include AGER; APP; CLEC12A; MICL; CTLA4; FOLR1; FZD1; FRIZZLED-1; KLRB1A; LRPAP1; NCR3; NKP30; OLR1; PROCR; PTPN1; SOX9; SCARB2; TACSTD2; TREM1; TREM2; TREML1; and VDR.

### COMPOSITIONS AND KITS

Aspects of the invention further include kits and compositions that find use in practicing various methods of the invention. Compositions include magnetic capture beads, e.g., as described above. In some instances, the magnetic capture beads may include barcode nucleic acids, e.g., as described above.

Kits may include magnetic captures beads, e.g., described above. Where the magnetic capture beads do not include barcode nucleic acids, the kits may further include barcoded beads, e.g., as described above. The kits may further include one or more additional components finding use in practicing embodiments of the methods. For example, the kits may include one or more of: primers, a polymerase (e.g., a thermostable polymerase, a reverse transcriptase both with hot-start properties, or the like), dsDNAse, exonuclease, dNTPs, a metal cofactor, one or more nuclease inhibitors (e.g., an RNase inhibitor and/or a DNase inhibitor), one or more molecular crowding agents (e.g., polyethylene glycol, or the like), one or more enzyme-stabilizing components (e.g., DTT), a stimulus response polymer, or any other desired kit component(s), such as devices, e.g., as described above, solid supports, containers, cartridges, e.g., tubes, beads, plates, microfluidic chips, etc. Components of the kits may be present in separate containers, or multiple components may be present in a single container.

In addition to the above components, the subject kits may further include (in certain embodiments) instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (e.g., bodies of the appended claims) are generally intended as "open" terms *(e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an"

(*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

## Claims

1. A method of barcoding nucleic acids of a particle, the method comprising:
a) combining a sample with a magnetic capture bead comprising a capture moiety for the particle of the sample to produce a captured sample, wherein barcode nucleic acids comprising a target binding region are tethered to the magnetic capture bead;
b) partitioning captured particles of the captured sample using an applied magnetic field mediated partitioning protocol to produce partitioned captured particles, and
c) lysing the partitioned captured particles so that nucleic acid acids released therefrom bind to the target binding regions of the bead bound barcode nucleic acids to produce captured nucleic acids.

2. The method according to claim 1 wherein the partitioned capture particles are partitioned into microwells.

3. The method according to any of the preceding claims, wherein the capture moiety comprises a specific binding member.

4. The method according to any of the preceding claims, wherein the bead bound barcode nucleic acids further comprise a cell label domain, a unique molecular index domain, and a universal primer binding domain.

5. The method according to any of the preceding claims, wherein the target binding region comprises an oligo dT domain, a gene specific domain or a random sequence domain.

6. The method according to any of the preceding claims, wherein the particle comprises a sub-cellular sized particle.

7. The method according to Claim 6, wherein the sub-cellular sized particle comprises a vesicle.

8. The method according to any of the preceding claims, wherein the method further comprises separating captured nucleic acids from other constituents of the partitioned captured particles.

9. The method according to Claim 8, wherein the separating comprises employing an applied magnetic field.

10. A magnetic capture bead comprising barcode nucleic acids tethered to the magnetic capture bead, wherein the barcode nucleic acids comprise a target binding region; and a capture moiety that specifically binds to a particle that can be lysed to release nucleic acids that bind to the target binding regions.

11. A device comprising:
a) a substrate comprising 100 or more microwells that comprise a magnetic capture bead comprising:
i) barcode nucleic acids tethered to the magnetic capture bead, wherein the barcode nucleic acids comprise a target binding region; and
ii) a capture moiety that specifically binds to a particle that can be lysed to release nucleic acids that bind to the target binding regions; and
b) a flow cell in fluid communication with the substrate.

12. A system comprising:
a) a substrate comprising 100 or more microwells that comprise a magnetic capture bead comprising:
i) barcode nucleic acids tethered to the magnetic capture bead, wherein the barcode nucleic acids comprise a target binding region; and
ii) a capture moiety that specifically binds to a particle that can be lysed to release nucleic acids that bind to the target binding regions;
b) a flow cell in fluid communication with the substrate; and
c) a flow controller; wherein the flow controller is configured to control the delivery of fluids to the flow cell.

13. A kit comprising a magnetic capture bead comprising barcode nucleic acids tethered to the magnetic capture bead, wherein the barcode nucleic acids comprise a target binding region, and wherein the magnetic capture bead also comprises a capture moiety that specifically binds to a target particle that can be lysed to release nucleic acids that bind to the target binding regions.

## Patentansprüche

1. Verfahren zum Barcoding von Nukleinsäuren eines Partikels, wobei das Verfahren umfasst:
a) Vereinigen einer Probe mit einem magnetischen Einfangkügelchen, das einen Einfangrest für das Partikel der Probe umfasst, um eine eingefangene Probe zu produzieren, wobei Barcode-Nukleinsäuren, die eine Zielbindungsregion umfassen, an das magnetische Einfangkügelchen gebunden werden;
b) Aufteilen der eingefangenen Partikel der eingefangenen Probe unter Verwendung eines durch ein angelegtes Magnetfeld vermittelten Aufteilungsprotokolls, um aufgeteilte eingefangene Partikel zu produzieren, und
c) Lysieren der aufgeteilten eingefangenen Partikel, sodass die daraus freigesetzten Nukleinsäuren an die Zielbindungsbereiche der an die Kügelchen gebundenen Barcode-Nukleinsäuren binden, um eingefangene Nukleinsäuren zu produzieren.

2. Verfahren nach Anspruch 1, wobei die aufgeteilten Einfangpartikel in Mikrovertiefungen aufgeteilt werden.

3. Verfahren nach einem der vorstehenden Ansprüche, wobei der Einfangrest ein spezifisches Bindungsglied umfasst.

4. Verfahren nach einem der vorstehenden Ansprüche, wobei die an die Kügelchen gebundenen Barcode-Nukleinsäuren ferner eine Zellmarkierungsdomäne, eine einzigartige molekulare Indexdomäne und eine universelle Primerbindungsdomäne umfassen.

5. Verfahren nach einem der vorstehenden Ansprüche, wobei die Zielbindungsregion eine Oligo-dT-Domäne, eine genspezifische Domäne oder eine Zufallssequenzdomäne umfasst.

6. Verfahren nach einem der vorstehenden Ansprüche, wobei das Partikel ein Partikel subzellulärer Größe umfasst.

7. Verfahren nach Anspruch 6, wobei das Partikel subzellulärer Größe ein Vesikel umfasst.

8. Verfahren nach einem der vorstehenden Ansprüche, wobei das Verfahren ferner Trennen der eingefangenen Nukleinsäuren von anderen Bestandteilen der aufgeteilten eingefangenen Partikel umfasst.

9. Verfahren nach Anspruch 8, wobei das Trennen Anwenden eines angelegten Magnetfelds umfasst.

10. Magnetisches Einfangkügelchen, umfassend an das magnetische Einfangkügelchen gebundene Barcode-Nukleinsäuren, wobei die Barcode-Nukleinsäuren eine Zielbindungsregion umfassen; und einen Einfangrest, der spezifisch an ein Partikel bindet, das lysiert werden kann, um Nukleinsäuren freizusetzen, die an die Zielbindungsregionen binden.

11. Vorrichtung, umfassend:
a) ein Substrat, umfassend 100 oder mehr Mikrovertiefungen, die ein magnetisches Einfangkügelchen umfassen, umfassend:
i) an die magnetische Einfangkügelchen gebundene Barcode-Nukleinsäuren, wobei die Barcode-Nukleinsäuren eine Zielbindungsregion umfassen; und
ii) einen Einfangrest, der spezifisch an ein Partikel bindet, das lysiert werden kann, um Nukleinsäuren freizusetzen, die an die Zielbindungsregionen binden;
und
b) eine Durchflusszelle in Fluidverbindung mit dem Substrat.

12. System, umfassend:
a) ein Substrat, umfassend 100 oder mehr Mikrovertiefungen, die ein magnetisches Einfangkügelchen umfassen, umfassend:
i) an die magnetische Einfangkügelchen gebundene Barcode-Nukleinsäuren, wobei die Barcode-Nukleinsäuren eine Zielbindungsregion umfassen; und
ii) einen Einfangrest, der spezifisch an ein Partikel bindet, das lysiert werden kann, um Nukleinsäuren freizusetzen, die an die Zielbindungsregionen binden;
b) eine Durchflusszelle in Fluidverbindung mit dem Substrat; und
c) einen Durchflussregler; wobei der Durchflussregler konfiguriert ist, um die Zufuhr von Fluiden zur Durchflusszelle zu steuern.

13. Kit, umfassend ein magnetisches Einfangkügelchen, das an das magnetische Einfangkügelchen gebundene Barcode-Nukleinsäuren umfasst, wobei die Barcode-Nukleinsäuren eine Zielbindungsregion umfassen, und wobei das magnetische Einfangkügelchen auch einen Einfangrest umfasst, der spezifisch an ein Zielpartikel bindet, das lysiert werden kann, um Nukleinsäuren freizusetzen, die an die Zielbindungsregionen binden.

## Revendications

1. Procédé de marquage par code-barres d'acides nucléiques d'une particule, le procédé comprenant :
a) la combinaison d'un échantillon avec une bille de capture magnétique comprenant une fraction de capture pour la particule de l'échantillon afin de produire un échantillon capturé, dans lequel les acides nucléiques de code-barres comprenant une région de liaison à la cible sont attachés à la bille de capture magnétique ;
b) le partitionnement des particules capturées de l'échantillon capturé à l'aide d'un protocole de partitionnement médié par un champ magnétique appliqué afin de produire des particules capturées partitionnées, et
c) la lyse des particules capturées partitionnées de manière à ce que les acides nucléiques libérés se lient aux régions de liaison à la cible des acides nucléiques de code-barres liés aux billes pour produire des acides nucléiques capturés.

2. Procédé selon la revendication 1, dans lequel les particules de capture partitionnées sont partitionnées dans des micropuits.

3. Procédé selon l'une quelconque des revendications précédentes, dans lequel la fraction de capture comprend un élément de liaison spécifique.

4. Procédé selon l'une quelconque des revendications précédentes, dans lequel les acides nucléiques de code-barres liés aux billes comprennent en outre un domaine d'étiquetage cellulaire, un domaine d'indice moléculaire unique et un domaine de liaison de l'amorce universelle.

5. Procédé selon l'une quelconque des revendications précédentes, dans lequel la région de liaison à la cible comprend un domaine oligo dT, un domaine spécifique à un gène ou un domaine de séquence aléatoire.

6. Procédé selon l'une quelconque des revendications précédentes, dans lequel particule comprend une particule de taille subcellulaire.

7. Procédé selon la revendication 6, dans lequel la particule de taille subcellulaire comprend une vésicule.

8. Procédé selon l'une quelconque des revendications précédentes, dans lequel le procédé comprend en outre la séparation des acides nucléiques capturés des autres constituants des particules capturées partitionnées.

9. Procédé selon la revendication 8, dans lequel la séparation comprend l'utilisation d'un champ magnétique appliqué.

10. Bille de capture magnétique comprenant des acides nucléiques de code-barres attachés à la bille de capture magnétique, dans laquelle les acides nucléiques de code-barres comprennent une région de liaison à la cible ; et une fraction de capture qui se lie spécifiquement à une particule qui peut être lysée pour libérer des acides nucléiques qui se lient aux régions de liaison à la cible.

11. Dispositif comprenant :
a) un substrat comprenant 100 micropuits ou plus qui comprennent une bille de capture magnétique comprenant :
i) des acides nucléiques de code-barres attachés à la bille de capture magnétique, dans lequel les acides nucléiques de code-barres comprennent une région de liaison à la cible ; et
ii) une fraction de capture qui se lie spécifiquement à une particule qui peut être lysée pour libérer des acides nucléiques qui se lient aux régions de liaison à la cible ;
et
b) une cellule à flux continu en communication fluidique avec le substrat.

12. Système comprenant :
a) un substrat comprenant 100 micropuits ou plus qui comprennent une bille de capture magnétique comprenant :
i) des acides nucléiques de code-barres attachés à la bille de capture magnétique, dans lequel les acides nucléiques de code-barres comprennent une région de liaison à la cible ; et
ii) une fraction de capture qui se lie spécifiquement à une particule qui peut être lysée pour libérer des acides nucléiques qui se lient aux régions de liaison à la cible ;
b) une cellule à flux continu en communication fluidique avec le substrat ; et
c) un régulateur de débit ; dans lequel le régulateur de débit est conçu pour commander l'acheminement de fluides vers la cellule à flux continu.

13. Kit comprenant une bille de capture magnétique comprenant des acides nucléiques de code-barres attachés à la bille de capture magnétique, dans lequel les acides nucléiques de code-barres comprennent une région de liaison à la cible, et dans lequel la bille de capture magnétique comprend également une fraction de capture qui se lie spécifiquement à une particule cible qui peut être lysée pour libérer des acides nucléiques qui se lient aux régions de liaison à la cible.
